# EUROPEAN PATENT SPECIFICATION

(11) **EP 2 539 303 B1**
(45) Date of publication and mention of the grant of the patent: **05.09.2018**
(21) Application number: 11704825.6
(22) Date of filing: 10.02.2011
(51) Int. Cl.: C07C 1/20, C07C 11/04, C07C 11/02, B01J 27/28, B01J 38/06, C07C 1/24

(54) **PROCESS FOR PREPARING AN ALKENE**
VERFAHREN ZUR HERSTELLUNG EINES ALKENS
PROCÉDÉ POUR LA PRÉPARATION D'UN ALCÈNE

(30) Priority: 24.02.2010 EP 10250327
(43) Date of publication of application: 02.01.2013
(73) Proprietor: Technip E&C Limited, Milton Keynes, Buckinghamshire MK9 1BA (GB)
(72) Inventor: PARTINGTON, Stephen, Roy, East Yorkshire HU12 8DS (GB)
(74) Representative: Stott, Michael John
(86) International application number: PCT/GB2011/000184
(87) International publication number: WO 2011/104494

(56) References cited:
- EP-A1- 0 384 755
- WO-A1-2008/062157
- WO-A2-02/102508
- JP-A- 2008 284 508

## Description

The present invention relates to the preparation of alkenes from oxygenates using supported heteropolyacid catalyst.

The present invention also relates to a process for treating supported heteropolytungstic acid catalysts useful in the preparation of alkenes from oxygenates.

Ethylene and other alkenes are important commodity chemicals and are useful starting materials for numerous chemical products, including polymeric products, such as polyethylene. Traditionally, alkenes, such as ethylene, have been produced by steam or catalytic cracking of hydrocarbons derived from crude oil. However, as crude oil is a finite resource, there is interest in finding alternative, economically viable, methods for producing alkenes, in particular ethylene, which can use feedstocks not derived from crude oil.

In recent years the search for alternative materials for alkene production has led to the production of alkenes by the dehydration of alcohols, such as methanol and ethanol, which can be produced by the fermentation of, for example, sugars, starches and/or cellulosic materials, or alternatively may be produced from synthesis gas.

US 5,177,114 discloses a process for the conversion of natural gas to gasoline grade liquid hydrocarbons and/or olefin(s) by converting the natural gas to a synthesis gas, and converting the synthesis gas to crude methanol and/or dimethyl ether and further converting the crude methanol/dimethyl ether to gasoline and olefin(s).

US 5,817,906 discloses a process for producing light olefin(s) from a crude oxygenate feedstock comprising alcohol and water. The process employs two reaction stages. Firstly, the alcohol is converted, using reaction with distillation, to an ether. The ether is then subsequently passed to an oxygenate conversion zone containing a metalaluminosilicate catalyst to produce a light olefin stream.

EP 1792885 discloses a process for the production of ethylene from a feedstock comprising ethanol. Catalysts based on heteropolyacids are disclosed as being suitable for the dehydration of the ethanol feedstock.

WO 2008/138775 A1 discloses a process for the dehydration of one or more alcohols, which process comprises contacting one or more alcohols in the presence of one or more ethers with a supported heteropolyacid catalyst.

US 4,398,050 describes the synthesis of a mixed alcohol stream and purification to give a mixture of ethanol and propanol, which is subsequently dehydrated at 0.05-0.1 MPa, 350-500°C (example 1). US 4,398,050 specifically discloses Al₂O₃, SiO₂, TiO₂, AlPO₄ and Ca₃(PO₄)₂ as examples of suitable dehydration catalysts, with alkalized aluminium oxide or calcium phosphate being disclosed as preferred catalysts.

It has been observed that dehydrating alcohols to produce alkenes, in particular the dehydration of ethanol to ethylene, can also result in the formation of alkanes. Alkenes of high purity are required for use in many chemical processes, such as in the production of polymers; therefore it may be necessary to remove alkanes from product alkene compositions prior to use. Removal of alkanes from alkenes, for example removal of ethane from product ethylene, can be very resource intensive and costly.

US 4,232,179 describes how ethanol can be dehydrated in adiabatic reactors. The examples, with silica /alumina, and alumina, show that the ethane content in the ethylene product is in the range of from 0.09 to 7.91 %wt.; this is unacceptable for polyethylene production without additional purification.

WO 2008/062157 A1 discloses a supported heteropolyacid catalyst; a process for producing alkenes from oxygenates in the presence of said catalyst; and, the use of said catalyst in a process for producing alkenes from oxygenates at a higher productivity whilst reducing the formation of alkanes.

WO 02/102508 A2 discloses a catalyst for use in the production of a lower aliphatic carboxylic acid ester by reacting a lower olefin and a lower aliphatic acid ester in a gas phase, which catalyst has been decreased in activity through use, is regenerated by heating to be recovered in activity. The regenerated catalyst is reused for the production of a lower aliphatic carboxylic acid ester.

JP 2008-284508 discloses a method for preparing a catalyst for use in producing methacrylic acid, said catalyst comprising a heteropolyacid compound containing phosphorus and molybdenum and said method comprising calcination in the presence of an oxidizing gas followed by calcination in the presence of a non-oxidizing gas.

The present invention provides an improved process for the production of an alkene from an oxygenate in the presence of a heteropolyacid catalyst; in particular, an improved process for the production of an alkene from an oxygenate in terms of alkane selectivity.

The present invention thus provides a process for the preparation of an alkene from an oxygenate comprising contacting a reactant feedstream comprising at least one oxygenate reactant and water with a supported heteropolyacid catalyst at a temperature of at least 170 °C, wherein the process is initiated using a start-up procedure comprising the following steps:
(i) heating the supported heteropolyacid catalyst to a temperature of at least 220 °C;
(ii) maintaining the heat-treated supported heteropolyacid catalyst of step (i) at a temperature of at least 220 °C for a time sufficient to remove bound water from the heteropolyacid component of the supported heteropolyacid catalyst; and
(iii) whilst maintaining the supported heteropolyacid catalyst of step (ii) at a temperature of at least 220 °C, contacting the supported heteropolyacid catalyst with the reactant feedstream having a temperature of at least 220 °C.

The supported heteropolyacid catalyst used in the process of the present invention may be a fresh catalyst or a spent and/or previously used catalyst, if the catalyst is a spent and/or previously used catalyst, prior to step (i) of the process, the supported heteropolyacid catalyst is preferably treated by heating the supported heteropolyacid catalyst to a temperature of at least 220 °C and passing steam over the heated supported heteropolyacid catalyst, followed by heating the steam-treated supported heteropolyacid catalyst to a temperature of at least 220 °C under an anhydrous atmosphere.

The present invention further provides a process for treating a supported heteropolytungstic acid catalyst for use in the preparation of an alkene from an oxygenate comprising the steps:
(a) heating the supported heteropolytungstic acid catalyst to a temperature of at least 220 °C and passing steam over said supported heteropolytungstic acid catalyst; and
(b) heating the supported heteropolytungstic acid catalyst treated in accordance with step (a) to at least 220 °C in an anhydrous atmosphere.

The supported heteropolyacid catalyst used in the preparation process of the present invention comprises a heteropolyacid supported on a suitable catalyst support.

The term "heteropolyacid", as used herein, refers to heteropolyacid compounds in the form of a free acid or in the form of a salt of the heteropolyacid, such as alkali metal salts, alkali earth metal salts, ammonium salts, bulky cation salts, and/or metal salts (where the salts may be either full or partial salts) of heteropolyacids.

The anion of the heteropolyacid typically comprises 12-18 oxygen-linked polyvalent metal atoms, known as the peripheral atoms, surrounding one or more of the central atom in a symmetrical manner. The peripheral atoms are suitably selected from molybdenum, tungsten, vanadium, niobium, tantalum, and combinations thereof. The central atoms are preferably silicon or phosphorus; alternatively, the central atoms may comprise any one of a large variety of atoms from Groups I-VIII in the Periodic Table of elements, such as copper, beryllium, zinc, cobalt, nickel, boron, aluminium, gallium, iron, cerium, arsenic, antimony, bismuth, chromium, rhodium, silicon, germanium, tin, titanium, zirconium, vanadium, sulphur, tellurium, manganese nickel, platinum, thorium, hafnium, tellurium and iodine. Suitable heteropolyacids include Keggin, Wells-Dawson and Anderson-Evans-Perloff heteropolyacids.

Preferably, the heteropolyacid component of the supported heteropolyacid catalyst is a heteropolytungstic acid, that is a heteropolyacid wherein the peripheral atoms are tungsten atoms. Preferred heteropolytungstic acids for use in the process of the present invention are any those based on the Keggin or Wells-Dawson structures.

Examples of suitable heteropolytungstic acids include: 18-tungstophosphoric acid (H₆[P₂W₁₈O₆₂].xH₂O); 12-tungstophosphoric acid (H₃[PW₁₂O₄₀].xH₂O); 12-tungstosilicic acid (H₄[SiW₁₂O₄₀].xH₂O); cesium hydrogen tungstosilicate (Cs₃H[SiW₁₂O₄₀].xH₂O); monopotassium tungstophosphate (KH₅[P₂W₁₈O₆₂].xH₂O); monosodium 12-tungstosilicic acid (NaK₃[SiW₁₂O₄₀].xH₂O); and, potassium tungstophosphate (K₆[P₂W₁₈O₆₂].xH₂O). Mixtures of two or more different heteropolytungstic acids and salts can also be used.

More preferably, the heteropolyacid component of the supported heteropolyacid catalyst is selected from silicotungstic acid, phosphotungstic acid, and mixtures thereof, for example, 12-tungstosilicic acid (H₄[SiW₁₂O₄₀].xH₂O), 12-tungstophosphoric acid (H₃[PW₁₂O₄₀].xH₂O), and mixtures thereof; even more preferably the heteropolyacid is a silicotungstic acid; most preferably the heteropolyacid is 12-tungstosilicic acid.

Preferably, the heteropolyacid employed in the present invention has molecular weight of more than 700 and less than 8500, preferably more than 2800 and less than 6000. Such heteropolyacids also include dimeric complexes thereof.

The hydration state of heteropolyacids can vary depending on various factors, such as temperature, and various hydration states for heteropolyacids are known. Typically, the hydration state of heteropolyacids decrease with increasing temperature; that is, the number of water molecules bound to the heteropolyacid decreases with increasing temperature. Thus, it is expected that the hydration state of the heteropolyacid component of the supported heteropolyacid catalyst used in the process of the present invention, before it has been subjected to the start-up procedure, is at least one; that is, the heteropolyacid component of the supported heteropolyacid catalyst has at least one water molecule bound thereto.

The supported heteropolyacid catalyst used in the process of the present invention may conveniently be prepared by first forming a heteropolyacid solution by dissolving a heteropolyacid in a suitable, typically polar, solvent, and then impregnating a suitable catalyst support with the heteropolyacid solution. Examples of suitable solvents include water, ethers, alcohols, carboxylic acids, ketones, aldehydes and mixtures thereof, with water, ethanol, and mixtures thereof, being preferred solvents.

The amount of heteropolyacid on the catalyst support is typically in the range of from 10 wt.% to 80 wt.% based on the weight of the supported heteropolyacid catalyst, preferably in the range of from 15 wt.% to 60 wt.%, more preferably in the range of from 20 wt.% to 50.wt. Preferably, the average heteropolyacid loading per surface area of the supported heteropolyacid catalyst is at least 0.1 micromoles/m².

The catalyst support used in the supported heteropolyacid catalyst may be any suitable catalyst support known in the art. Examples of suitable materials for the catalyst support include mordenites (e.g. montmorillonite), clays, bentonite, diatomous earth, titania, activated carbon, alumina, silica, silica-alumina, silica-titania cogels, silica-zirconia cogels, carbon coated alumina, zeolites, zinc oxide, and flame pyrolysed oxides. Catalyst supports based on silica are preferred, such as silica gel supports and supports produced by the flame hydrolysis of SiCl₄.

The shape of the catalyst support is not critical to the present invention, for example the catalyst support may be in a powder form, a granular form, a pelletised form, a spherical form, or in the form of an extrudate.

Examples of suitable catalysts and catalyst support materials that may be used in the supported heteropolyacid catalysts, as well as the preparations of said catalysts and supports, are described in WO 2008/062157 A1.

The reactant feedstream used in the process of the present invention comprises at least one oxygenate reactant and water.

Preferably, the oxygenate reactant component of the reactant feedstream, also referred to herein as the oxygenate reactant(s), used in the process of the present invention is an alcohol and/or an alcohol derivative. Preferred alcohol derivatives that may be used in the process of the present invention are ethers; thus the oxygenate reactant(s) used in the process of the present invention is preferably an alcohol and/or an ether derivative thereof. Preferably, the alcohol(s) and/or derivative(s) thereof in the oxygenate reactant(s) of the process of the present invention are monohydric aliphatic alcohols having from two to six carbon atoms and/or ether derivatives thereof. More preferably, the oxygenate reactant(s) of the process of the present invention are selected from ethanol, propanol, isopropanol, n-butanol, t-butanol, diethyl ether, dipropyl ether, diisopropyl ether, di-n-butyl ether, di-t-butyl ether, ethoxypropane, ethoxyisopropane, ethoxy-n-butane, ethoxy-t-butane, propoxyisopropane, propoxy-n-butane, propoxy-t-butane, isopropoxy-n-butane, isopropoxy-t-butane, n-butoxy-t-butane and mixtures thereof. Even more preferably, the oxygenate reactant(s) of the process of the present invention is ethanol and/or derivatives thereof, in particular ethanol and/or diethyl ether. Most preferably, the oxygenate reactants of the process of the present invention are ethanol and diethyl ether, i.e. the reactant feedstream used in the process of the present invention comprises ethanol, diethyl ether and water.

In a particular embodiment of the present invention, the oxygenate reactant component of the reactant feedstream used in the process of the present invention is an oxygenate composition comprising at least 95 wt.% ethanol and/or diethyl ether, based on the total amount of oxygenates, more preferably at least 98 wt.% ethanol and/or diethyl ether, most preferably at least 99.5 wt.% ethanol and/or diethyl ether.

Preferably, the amount of water in the reactant feedstream of the process of the present invention is at most 50 wt.%, more preferably at most 20 wt.%, most preferably at most 10 wt.%, or even at most 5 wt.%, based on the total weight of water and oxygenate in the reactant feedstream. Preferably, the amount of water in the reactant feedstream is at least 0.1 wt.%, more preferably at least 0.5 wt.% and most preferably at least 1 wt.%, based on the total weight of water and oxygenate in the reactant feedstream.

According to a preferred embodiment of the present invention, the operating conditions under which the dehydration process is conducted are such that the dehydration process is always operated in a vapour phase state.

The temperature at which the dehydration process according to the present invention (the process for the preparation of an alkene from an oxygenate) is conducted is at least 170 °C, preferably in the range of from 180 to 270 °C, more preferably in the range of from 190 to 260 °C and most preferably in the range of from 200 to 250 °C.

The pressure at which the dehydration process according to the present invention (the process for the preparation of an alkene from an oxygenate) is conducted is preferably a pressure in the range of from 0.1 MPa to 4.5 MPa, more preferably at a pressure in the range of from 1.0 MPa to 3.5 Mpa, and most preferably at a pressure in the range of from 1.0 MPa to 2.8 MPa.

The product composition of the process of the present invention typically comprises alkenes, unreacted oxygenate reactant(s) (e.g. alcohols), ethers, water and alkanes.

Typically, the alkenes are separated from the product composition and the unreacted oxygenate reactant(s) (e.g. alcohols) and ethers are preferably recycled back to the process of the present invention. Typically, at least part of the water of the product composition is also recycled back to the process of the present invention together with the unreacted oxygenate reactant(s) and ethers.

Because alkenes and their corresponding alkanes have relatively close boiling points, the alkene composition which is separated from the product composition often contains the corresponding alkanes that have been produced. Therefore, minimising the amount of alkanes produced during the preparation of alkenes from oxygenates is highly desirable.

It has been unexpectedly found that the amount of alkanes produced during the process for preparing an alkene from an oxygenate comprising contacting a reactant feedstream comprising at least one oxygenate reactant and water with a supported heteropolyacid catalyst at a temperature of at least 170 °C, varies depending upon the way in which the process is initiated. Therefore, by initiating the process using a the start-up procedure described herein, it is possible to provide a process where the amount of alkanes produced is controlled at a low level relative to processes initiated using a supported heteropolyacid catalyst wherein said catalyst has not been subjected to a treatment to remove bound water from the heteropolyacid component of the supported heteropolyacid catalyst.

Thus, the present invention provides a process for the preparation of an alkene from an oxygenate comprising contacting a reactant feedstream comprising at least one oxygenate reactant and water with a supported heteropolyacid catalyst at a temperature of at least 170 °C, wherein the process is initiated using a start-up procedure comprising the following steps:
(i) heating the supported heteropolyacid catalyst to a temperature of at least 220 °C;
(ii) maintaining the heat-treated supported heteropolyacid catalyst of step (i) at a temperature of at least 220 °C for a time sufficient to remove bound water from the heteropolyacid component of the supported heteropolyacid catalyst; and
(iii) whilst maintaining the supported heteropolyacid catalyst of step (ii) at a temperature of at least 220 °C, contacting the supported heteropolyacid catalyst with the reactant feedstream having a temperature of at least 220 °C.

Due to the nature of heteropolyacids, the process for preparing supported heteropolyacid catalysts, and the loading of said catalysts into a reaction zone, the heteropolyacid component will almost certainly be exposed to water (such as moisture in the atmosphere) under conditions at which it may become bound to the heteropolyacid component, and thus the hydration state of the heteropolyacid component of the supported heteropolyacid catalyst prior to heating the supported heteropolyacid catalyst in step (i) of the start-up procedure will be above zero (i.e. the heteropolyacid component of the supported heteropolyacid catalyst has water molecules chemically bound thereto). Thus, in the process of the present invention, the supported heteropolyacid catalyst prior to being subjected to the start-up procedure of the present invention is a supported heteropolyacid catalyst wherein the heteropolyacid component thereof has a hydration state above zero.

Whilst not wishing to be bound by theory, it is believed that by performing steps (i) and (ii) of the start-up procedure described above, water that is bound to the heteropolyacid component of the supported heteropolyacid catalyst is removed, and that at least part of the heteropolyacid component of the supported heteropolyacid catalyst is reduced to being in the zero hydration state (i.e. the heteropolyacid component having no bound water molecules). Therefore, by the term "remove bound water from the heteropolyacid component of the supported heteropolyacid catalyst" it is meant that at least part of the heteropolyacid component of the supported heteropolyacid catalyst has had its hydration state reduced to zero; more preferably at least 50 %wt. of the supported heteropolyacid catalyst has had its hydration state reduced to zero; most preferably at least 75 %wt. of the supported heteropolyacid catalyst has had its hydration state reduced to zero. Thus, at least part of the heteropolyacid component of the supported heteropolyacid catalyst has a zero hydration state (having no bound water molecules) when it is contacted with the oxygenate reactant or the reactant feedstream.

Whilst the process to prepare alkenes from oxygenates using a supported heteropolyacid catalyst can be performed under conditions which would lead to/maintain a hydration state of the heteropolyacid component of one or more (i.e. the heteropolyacid component having at least one bound water molecule), it is believed that the propensity for the process to produce alkanes is increased with the increasing amount of the heteropolyacid component that is not in the zero hydration state during the initiation of the process.

Therefore, once the process to prepare alkenes from oxygenates using a supported heteropolyacid catalyst has been initiated using the start-up procedure described above, the reaction temperature can be adjusted to a temperature below 220 °C without significantly increasing the amount of alkane(s) produced.

Preferably, either or both of step (i) and step (ii) of the above-described start-up procedure is performed under a stream of inert gas. By the term "inert gas" as used herein, it is meant a gas that is not consumed in the reaction of the process of the present invention, and is not consumed by any other process which may be catalysed by the supported heteropolyacid catalyst. Examples of suitable inert gases are nitrogen, argon, helium, methane and carbon dioxide. Preferably, the inert gas is selected from nitrogen, argon and helium, more preferably, the inert gas is nitrogen. By the term "stream of inert gas" as used herein, it is meant that the atmosphere under which the step takes place is an inert gas that is constantly being removed and replenished with fresh (or recycled) inert gas (i.e. a gas flow). For example, the "stream of inert gas" is preferably a stream of nitrogen gas.

Therefore, step (i) and/or step (ii) of the above-described start-up procedure are preferably performed under a stream of nitrogen gas.

The temperature to which the supported heteropolyacid catalyst is heated in step (i) and maintained at in step (ii) of the above-described start-up procedure is at least 220 °C. Higher temperatures may be used as this can increase the rate at which bound water is removed from the heteropolyacid component of the supported heteropolyacid catalyst. Thus, it is preferred that the temperature to which the supported heteropolyacid catalyst is heated in step (i) and maintained at in step (ii) of the above-described start-up procedure is greater than 220 °C; for instance, temperatures of at least 230 °C, at least 240 °C, or even at least 250 °C, can conveniently be used. In a preferred embodiment of the present invention, the temperature to which the supported heteropolyacid catalyst is heated in step (i) and maintained at in step (ii) of the start-up procedure is at least 240 °C. Preferably, the temperature to which the supported heteropolyacid catalyst is heated in step (i) and maintained at in step (ii) of the start-up procedure is at most 450 °C, more preferably at most 400 °C, even more preferably at most 350 °C.

The amount of time the supported heteropolyacid catalyst is maintained at a temperature of at least 220 °C in step (ii) of the start-up procedure is sufficient to remove at least a portion of, preferably most of, more preferably all of, the bound water from the heteropolyacid component of the supported heteropolyacid catalyst. Because the removal of bound water from the heteropolyacid component of a supported heteropolyacid catalyst is endothermic, the skilled person will be able to determine when such a process is occurring and/or when the removal of bound water from the heteropolyacid component of a supported heteropolyacid catalyst is complete by monitoring the heat flow and weight loss of the catalyst during step (i) and step (ii) of the start-up procedure; or, when step (i) and step (ii) are performed under a stream of inert gas, by monitoring the amount of water present in the exit gas flow.

Preferably, step (ii) is conducted for sufficient time such that the removal of water from the heteropolyacid component can no longer be detected.

In one embodiment of the present invention, the amount of time that the supported heteropolyacid catalyst is maintained at a temperature of at least 220 °C in step (ii) of the start-up procedure is at least 1 hour, preferably at least 2 hours, more preferably at least 5 hours, even more preferably at least 10 hours, most preferably at least 20 hours.

Because higher temperatures can increase the rate at which bound water is removed from the heteropolyacid component of the supported heteropolyacid catalyst, it is preferable to maintain the heat-treated supported heteropolyacid catalyst of step (i) at the temperature of at least 220 °C in step (ii) for a longer duration when lower temperatures are used compared to when higher temperatures are used. Thus, in one specific embodiment of the present invention, in step (ii), the heat-treated supported heteropolyacid catalyst of step (i) is preferably maintained at a temperature of at least 220 °C for at least 10 hours, more preferably at least 20 hours. In another specific embodiment of the present invention, in step (ii), the heat-treated supported heteropolyacid catalyst of step (i) is preferably maintained at a temperature of at least 230 °C for at least 5 hours, more preferably at least 10 hours. In yet another specific embodiment of the present invention, in step (ii), the heat-treated supported heteropolyacid catalyst of step (i) is preferably maintained at a temperature of at least 240 °C for at least 2 hours, more preferably at least 5 hours. In yet another specific embodiment of the present invention, in step (ii), the heat-treated supported heteropolyacid catalyst of step (i) is preferably maintained at a temperature of at least 250 °C for at least 1 hour, more preferably at least 2 hours.

Whilst not wishing to be bound by theory, it is believed that at temperatures of at least 220 °C, any water present in the atmosphere in which the supported heteropolyacid catalyst is present will not become bound to the heteropolyacid component of the catalyst and will not prevent the water that may already be bound to the heteropolyacid component from being removed.

Therefore, both step (i) and step (ii) of the start-up procedure may be performed under a hydrous or anhydrous atmosphere. By the term "anhydrous atmosphere" it is meant an atmosphere which would be considered by the skilled person as containing essentially no water in respect of the process of the present invention; preferably, by the term "anhydrous atmosphere", as used herein, it is meant an atmosphere which contains no more than 5 ppmv water. By the term "hydrous atmosphere" it is meant an atmosphere which would be considered by the skilled person as containing water in respect of the process of the present invention; preferably, by the term "hydrous atmosphere", as used herein, it is meant an atmosphere which contains more than 5 ppmv water.

The use of an anhydrous atmosphere is not essential as it has been found that the presence of water during the above-described start-up procedure does not significantly change the amount of alkane produced during the process for preparing alkenes from oxygenates using a fresh supported heteropolyacid catalyst compared to when the above-described start-up procedure is performed under an anhydrous atmosphere.

In a preferred embodiment of the present invention, step (i) and step (ii) of the start-up procedure are performed under an anhydrous atmosphere.

However, since it is believed that water will not become bound to the heteropolyacid component at temperatures of at least 220 °C, in an alternative embodiment of the present invention, in particular when a fresh supported heteropolyacid catalyst is used, step (i) and/or step (ii) of the start-up procedure are performed in the presence of water; for example step (i) may be performed under anhydrous conditions and step (ii) may be performed in the presence of water, or vice versa.

By the term "fresh supported heteropolyacid catalyst", it is meant a supported heteropolyacid catalyst that has not previously been employed as a catalyst in any reaction, i.e. not a spent or regenerated catalyst. By the term "regenerated" when used in relation to a supported heteropolyacid catalyst, it is meant that a supported heteropolyacid catalyst whose efficiency in the process of the present invention is lower than desired which has subsequently been treated to increase the efficiency of the catalyst in the process of the present invention. The term "efficiency in the process of the present invention" is used to encompass one or more of the catalyst activity, the alkene selectivity, and the alkane selectivity. Independently, a high catalyst activity is desirable in the process of the present invention; a high alkene selectivity is desirable in the process of the present invention; and, a low alkane selectivity is desirable in the process of the present invention.

In yet another alternative embodiment of the present invention, step (i) and step (ii) of the start-up procedure are initially performed under an anhydrous atmosphere, followed by the addition of water before step (iii) of the start-up procedure.

The contacting of the supported heteropolyacid catalyst with the reactant feedstream in step (iii) of the start up procedure can optionally be performed in a step-wise manner, for instance, by initially contacting the water component of the reactant feedstream with the supported heteropolyacid catalyst followed by addition of the oxygenate reactant(s) to the water component to form the reactant feedstream, or vice versa.

In a preferred embodiment of the present invention, step (iii) of the start-up procedure is performed in two steps:
(iiia) whilst maintaining the supported heteropolyacid catalyst of step (ii) at a temperature of at least 220 °C, contacting the supported heteropolyacid catalyst with water having a temperature of at least 220 °C; and
(iiib) whilst maintaining the supported heteropolyacid catalyst of step (iiia) at a temperature of at least 220 °C, introducing the oxygenate reactant to the water of step (iiia) to form the reactant feedstream

Prior to employing the supported heteropolyacid catalyst in the process for the preparation of an alkene from an oxygenate of the present invention, the supported heteropolyacid catalyst can optionally be treated by heating to a temperature of at least 220 °C and passing steam over the heated supported heteropolyacid catalyst, followed by heating the steam-treated supported heteropolyacid catalyst to a temperature of at least 220 °C under an anhydrous atmosphere. Preferably, the initial heating of the supported heteropolyacid catalyst to a temperature of at least 220 °C in this optional treatment is performed under an anhydrous atmosphere.

This optional treatment of the supported heteropolyacid catalyst can conveniently be performed prior to step (i) of the start-up procedure. Alternatively, the optional treatment of the supported heteropolyacid catalyst can be performed during steps (i) and (ii) of the start-up procedure. In such an embodiment, step (i) is performed under an anhydrous atmosphere, and during step (ii) steam is passed over the heated supported heteropolyacid catalyst followed by maintaining the catalyst at a temperature of least 220 °C under an anhydrous atmosphere.

Preferably, the anhydrous atmosphere for the start-up procedure or for the optional treatment of the supported heteropolyacid catalyst is an anhydrous, inert gas atmosphere, more preferably a stream of inert gas; typically, the anhydrous atmosphere is a stream of nitrogen gas.

This optional treatment of the supported heteropolyacid catalyst prior to employing said catalyst in the process for the preparation of an alkene from an oxygenate of the present invention can be performed on either a fresh catalyst or a catalyst that has been previously used in the process for the preparation of an alkene from an oxygenate. In particular, it has been found that this optional treatment of the supported heteropolyacid catalyst prior to employing said catalyst in the process of the present invention is particularly beneficial when the supported heterogeneous catalyst to be used in the process of the present invention has previously been employed in a process for the preparation of an alkene from an oxygenate; in particular, by using this optional treatment on a supported heterogeneous catalyst which has previously been employed in a process for the preparation of an alkene from an oxygenate, the alkane selectivity of the catalyst is lower than when this optional treatment has not been performed.

Thus, by use of this optional treatment of the supported heteropolyacid catalyst prior to employing said catalyst in the process for the preparation of an alkene from an oxygenate of the present invention, a previously used supported heteropolyacid catalyst may be regenerated; in particular, the alkane selectivity of the catalyst can be reduced compared to the alkane selectivity of the catalyst prior to the above-described optional treatment.

Whilst not wishing to be bound by theory, it is believed that this optional treatment of the supported heteropolyacid catalyst removes more contaminants from the supported heteropolyacid catalyst than would be removed by passing nitrogen gas over the catalyst only or by passing steam over the catalyst only.

Therefore, the present invention further provides a process for treating a supported heteropolytungstic acid catalyst for use in the preparation of an alkene from an oxygenate comprising the steps:
(a) heating the supported heteropolytungstic acid catalyst to a temperature of at least 220 °C and passing steam over said supported heteropolytungstic acid catalyst; and
(b) heating the supported heteropolytungstic acid catalyst treated in accordance with step (a) to at least 220 °C in an anhydrous atmosphere.

Preferably, the initial heating of the supported heteropolytungstic acid catalyst to a temperature of at least 220 °C in step (a) is performed under an anhydrous atmosphere.

Preferably, step (b) of this process for treating a supported heteropolytungstic acid catalyst is performed directly after step (a) whilst maintaining the catalyst at a temperature of at least 220 °C throughout the entire process. Therefore, the process for treating a supported heteropolytungstic acid catalyst preferably comprises the steps:
(a') heating the supported heteropolytungstic acid catalyst to a temperature of at least 220 °C under an anhydrous atmosphere;
(b') whilst maintaining the supported heteropolytungstic acid catalyst at a temperature of at least 220 °C, passing steam over said supported heteropolytungstic acid catalyst;
(c') whilst maintaining the supported heteropolytungstic acid catalyst at a temperature of at least 220 °C, ceasing passing steam over said supported heteropolytungstic acid catalyst; and
(d') maintaining the supported heteropolytungstic acid catalyst at a temperature of at least 220 °C in an anhydrous atmosphere.

Preferably, step (b') of the above process is performed for at least 30 minutes, more preferably at least 1 hour. Preferably, step (d') of the above process is performed for at least 30 minutes, more preferably at least 1 hour, even more preferably for at least 2 hours.

The above process may be performed prior to step (i) of the start-up procedure described herein, or may alternatively be performed during steps (i) and (ii) of the start-up procedure.

Thus, in a preferred embodiment, the above process for treating a supported heteropolytungstic acid catalyst comprises heating the supported heteropolytungstic acid catalyst to a temperature of at least 220 °C under a nitrogen atmosphere, whilst maintaining the catalyst at a temperature of at least 220 °C, passing steam over the catalyst, preferably for at least one hour, followed by passing a stream of nitrogen gas over the catalyst.

Once the supported heteropolytungstic acid catalyst has been treated as described above, it may then be subjected directly to the process of the present invention without first cooling the catalyst to a temperature of below 220 °C, or may first be cooled to a temperature of below 220 °C.

Conveniently, when the supported heteropolytungstic acid catalyst which is to be treated by a process as described above is a spent catalyst, or a catalyst that has previously been employed in a process for the preparation of an alkene from an oxygenate, then treatment of the supported heteropolytungstic acid catalyst by the process described above may be performed prior to removing the catalyst from a reactor and/or disposing of the catalyst.

The present invention yet further provides a process for the preparation of an alkene from an oxygenate comprising contacting a reactant feedstream comprising at least one oxygenate reactant and water with a supported heteropolytungstic acid catalyst at a temperature of at least 170 °C, wherein the supported heteropolytungstic acid catalyst is treated in accordance with the process for treating a supported heteropolytungstic acid catalyst comprising steps (a) and (b) described herein, preferably in accordance with the process for treating a supported heteropolytungstic acid catalyst comprising steps (a'), (b'), (c') and (d') described hereinabove, and the process for the preparation of an alkene from an oxygenate is initiated using a start-up procedure comprising steps (i), (ii) and (iii) described hereinabove.

Therefore, the present invention yet further provides a process for the preparation of an alkene from an oxygenate comprising contacting a reactant feedstream comprising at least one oxygenate reactant and water with a supported heteropolytungstic acid catalyst at a temperature of at least 170 °C, wherein the supported heteropolytungstic acid catalyst has previously been used in a process for the preparation of an alkene from an oxygenate and has been regenerated by the process for treating a supported heteropolytungstic acid catalyst described hereinabove, and wherein the process for the preparation of an alkene from an oxygenate is initiated by the start-up procedure described hereinabove.

The present invention further provides the use of the above described start-up procedure for a process for producing alkenes from oxygenates using a supported heteropolyacid catalyst, for reducing the amount of alkanes produced relative to a corresponding process which was initiated using a start-up procedure which did not comprise both step (i) and step (ii).

### Examples

The following examples were all performed in a micro-reactor having an internal diameter of 15 mm, a length of 69 cm, and having a 5mm (outside diameter) thermowell inserted in the reactor in the axial direction. The thermowell inserted in the reactor contained four thermocouples with the first being placed in a pre-heat zone where the liquid feed is vapourised, and the other three being placed in the catalyst bed. The pressure of the process was controlled by a pressure control valve (PCV) with all vapours exiting the reactor passing to the low pressure side of the PCV. A portion of the exit gas was directed to a GC for on-line analysis of the products.

In all the examples, approximately 2.7g of the catalyst, which is equivalent to a bulk volume of 5cm³, was loaded into the reactor. The catalyst was also mixed with an inert diluent of Davicat (trademark) A372 (also known as G57) silica (2.7g, which was of 0.25 to 0.5mm diameter). The diluent was used to fill the voids between the catalyst particles allowing good interaction of the reactants with the catalyst (i.e. no channelling).

### Examples 1 and 2

The catalyst used in Examples 1 and 2 was silicotungstic acid (12-tungstosilicic acid) (ex. Nippon Inorganic Chemicals) supported on CariAct (trademark) Q15 silica pellets (ex. Fuji Silysia) at a concentration of 275g/kg silicotungstic acid.

In Examples 1 and 2, the reactant feedstream detailed in Table 1 was used.

**Table 1.**

| Liquid Feed | |
|---|---|
| Ethanol (%wt) | 33.00 |
| Diethyl ether (%wt) | 65.50 |
| Water (%wt) | 1.50 |

| Feed Rate | |
|---|---|
| Liquid Feed Rate (g/min) | 0.377 |
| Nitrogen (g/min) | 0.1150 |

In Example 1, a fresh catalyst was heated to a temperature of 250 °C under a flow of nitrogen (20 barg: 0.115 g/min) and maintained at 250 °C under the nitrogen stream for 2 hours. The temperature was then reduced to 220 °C. Once the temperature of the catalyst was at 220 °C, the reactant feedstream detailed in Table 1 was introduced to the reactor at a pressure of 20 barg and these conditions were maintained for 90 minutes. The temperature was then increased to 240 °C and the pressure was increased to 30 barg over ten minutes and the reactor was maintained under these conditions. The performance of the catalyst in the preparation of ethylene from the reactant feedstream detailed in Table 1 is provided by the product composition after 66 hours on stream recorded in Table 2 below.

In Example 2, a fresh catalyst was heated to a temperature of 180 °C under a flow of nitrogen (20 barg: 0.115 g/min) and maintained at 180 °C under the nitrogen stream for 30 minutes. The reactant feedstream detailed in Table 1 was then introduced to the reactor at a pressure of 20 barg and these conditions were maintained for 2 hours. The temperature was then increased to 240 °C and the pressure was increased to 30 barg over ten minutes and the reactor was maintained under these conditions. The performance of the catalyst in the preparation of ethylene from the reactant feedstream detailed in Table 1 is provided by the product composition after 85 hours on stream recorded in Table 2 below.

**Table 2**

| Example | Ethylene Space Time Yield (g/l/hr) | Ethane (ppmw on ethylene product) | C₄* (ppmw on ethylene product) | Acetaldehyde (ppmw on ethylene product) |
|---|---|---|---|---|
| 1 | 863 | 318 | 1063 | 552 |
| 2^ | 877 | 600 | 3384 | 1912 |

| | | | | |
|---|---|---|---|---|
| * - Hydrocarbons containing four carbon atoms, primarily butenes. ^ - Not of the invention. | | | | |

As can be seen from the results presented in Table 2, the concentration of ethane present in the product composition when the process was started using the process of the present invention is significantly lower than when the process was started up using a lower temperature.

### Examples 3 and 4

The catalyst used in Examples 3 and 4 was silicotungstic acid (12-tungstosilicic acid) (ex. Nippon Inorganic Chemicals) supported on CariAct (trademark) Q15 silica pellets (ex. Fuji Silysia) at a concentration of silicotungstic acid equivalent to 22.4 %w/w tungsten (by analysis).

The tungsten analysis was achieved by: i) drying the catalyst at 130 °C for 3 hours; ii) shaking a known weight of the dried sample in a known volume of water and determining the tungsten in the aqueous filtrate by Inductively Coupled Plasma Optical Emission Spectroscopy (ICP); iii) taking the remaining residue, ashing at 550 °C, treating the remaining solid with hydrofluoric acid followed by fusion with lithium borate flux, dissolution in an acidic solution and determining the tungsten in the acidic solution by ICP. The sum of the individual tungsten analyses from (ii) and (iii) gave the total tungsten in the catalyst.

For Examples 3 and 4, the reactant feedstream detailed in Table 3 was used.

**Table 3.**

| Liquid Feed | |
|---|---|
| Ethanol (%wt) | 47.6 |
| Diethyl ether (%wt) | 48.1 |
| Water (%wt) | 4.3 |

| Feed Rate | |
|---|---|
| Liquid Feed Rate (g/min) | 0.387 |
| Nitrogen (g/min) | 0.0925 |

In Example 3, a fresh catalyst was heated to a temperature of 220°C under a flow of nitrogen (20 barg: 0.115 g/min) and maintained at 220 °C under the nitrogen stream for 24 hours. The reactant feedstream detailed in Table 3 was introduced to the reactor at a pressure of 20 barg and these conditions were maintained for approximately 10 minutes. The temperature was then increased to 240 °C and the pressure was increased to 30 barg over approximately 10 minutes. The temperature was subsequently increased to 250 °C and the flow of nitrogen reduced to 0.0925g/min and the reactor was maintained under these conditions. The performance of the catalyst in the preparation of ethylene from the reactant feedstream detailed in Table 3 is provided by the product composition after 115 hours on stream recorded in Table 4 below.

In Example 4, a fresh catalyst was heated to a temperature of 220°C under a flow of nitrogen (20 barg: 0.115 g/min) and maintained at 220 °C under the nitrogen stream for 2 hours. The reactant feedstream detailed in Table 3 was introduced to the reactor at a pressure of 20 barg and these conditions were maintained for approximately 10 minutes. The temperature was then increased to 240 °C and the pressure was increased to 30 barg over approximately 10 minutes. The temperature was subsequently increased to 250 °C and the flow of nitrogen reduced to 0.0925g/min and the reactor was maintained under these conditions. The performance of the catalyst in the preparation of ethylene from the reactant feedstream detailed in Table 3 is provided by the product composition after 115 hours on stream recorded in Table 4 below.

**Table 4.**

| Example | Ethylene Space Time Yield (g/l/hr) | Ethane (ppmw on ethylene product) | C₄* (ppmw on ethylene product) | Acetaldehyde (ppmw on ethylene product) |
|---|---|---|---|---|
| 3 | 991 | 353 | 1415 | 686 |
| 4 | 927 | 525 | 2430 | 897 |

| | | | | |
|---|---|---|---|---|
| * - Hydrocarbons containing four carbon atoms, primarily butenes | | | | |

As can be seen from the results presented in Table 4, the concentration of ethane, and other by-products, present in the product composition is reduced when the catalyst is maintained at the temperature of at least 220 °C for a longer duration.

## Claims

1. A process for the preparation of an alkene from an oxygenate comprising contacting a reactant feedstream comprising at least one oxygenate reactant and water with a supported heteropolyacid catalyst at a temperature of at least 170 °C, wherein the process is initiated using a start-up procedure comprising the following steps:
(i)heating the supported heteropolyacid catalyst to a temperature of at least 220 °C;
(ii)maintaining the heat-treated supported heteropolyacid catalyst of step (i) at a temperature of at least 220 °C for a time sufficient to remove bound water from the heteropolyacid component of the supported heteropolyacid catalyst; and
(iii)whilst maintaining the supported heteropolyacid catalyst of step (ii) at a temperature of at least 220 °C, contacting the supported heteropolyacid catalyst with the reactant feedstream having a temperature of at least 220 °C.

2. Process according to claim 1, wherein in step (ii), the heat-treated supported heteropolyacid catalyst of step (i) is maintained at a temperature of at least 220 °C for at least one hour.

3. Process according to claim 1 or claim 2, wherein step (iii)is performed in two steps:
(iiia) whilst maintaining the supported heteropolyacid catalyst of step (ii) at a temperature of at least 220 °C, contacting the supported heteropolyacid catalyst with water having a temperature of at least 220 °C; and
(iiib) whilst maintaining the supported heteropolyacid catalyst of step (iiia) at a temperature of at least 220 °C, introducing the oxygenate reactant to the water of step (iiia) to form the reactant feedstream

4. Process according to any one of claims 1 to 3, wherein the oxygenate reactant(s) is an alcohol and/or alcohol derivative.

5. Process according to claim 4, wherein the oxygenate reactant(s) is ethanol and/or a derivative of ethanol.

6. Process according to claim 5, wherein the oxygenate reactant is ethanol.

7. Process according to any one of claims 1 to 6, wherein the supported heteropolyacid catalyst is a supported silicotungstic acid catalyst.

8. Process according to claim 7, wherein the supported heteropolyacid catalyst is a supported 12-tungstosilicic acid catalyst.

9. Process according to any one of claims 1 to 8, wherein the amount of heteropolyacid in the supported heteropolyacid catalyst is in the range of from 10 wt.% to 50 wt.% based on the total weight of the supported heteropolyacid catalyst.

10. Process according to any one of claims 1 to 9, wherein the process for the preparation of an alkene from an oxygenate is performed at a temperature in the range of from 180 °C to 270 °C.

11. Process according to any one of claims 1 to 10, wherein the process for the preparation of an alkene from an oxygenate is performed at a pressure in the range of from 0.1 MPa to 4.5 MPa.

12. Process according to any one of claims 1 to 11, wherein prior to step (i) of the process, the supported heteropolyacid catalyst is preferably treated by heating the supported heteropolyacid catalyst to a temperature of at least 220 °C and passing steam over the heated supported heteropolyacid catalyst, followed by heating the steam-treated supported heteropolyacid catalyst to a temperature of at least 220 °C under an anhydrous atmosphere.

13. Process according to claim 12, wherein the supported heteropolyacid catalyst has previously been employed in a process for the preparation of an alkene from an oxygenate.

14. A process for treating a supported heteropolytungstic acid catalyst for use in the preparation of an alkene from an oxygenate comprising the steps:
(a) heating the supported heteropolytungstic acid catalyst to a temperature of at least 220 °C and passing steam over said supported heteropolytungstic acid catalyst; and
(b) heating the supported heteropolytungstic acid catalyst treated in accordance with step (a) to at least 220 °C in an anhydrous atmosphere.

15. A process according to claim 14, wherein step (b) is performed directly after step (a) whilst maintaining the catalyst at a temperature of at least 220 °C throughout the entire process.

## Patentansprüche

1. Verfahren zur Herstellung eines Alkens aus einem Oxygenat, umfassend das Inkontaktbringen eines Reaktantenzulaufs, umfassend mindestens einen Oxygenatreaktanten und Wasser, mit einem geträgerten Heteropolysäure-Katalysator bei einer Temperatur von mindestens 170 °C, wobei das Verfahren mit einem Anfahrvorgang initiiert wird, umfassend die folgenden Schritte:
(i) Erwärmen des geträgerten Heteropolysäure-Katalysators auf eine Temperatur von mindestens 220 °C;
(ii) Halten des wärmebehandelten geträgerten Heteropolysäure-Katalysators von Schritt (i) bei einer Temperatur von mindestens 220 °C für eine Zeitdauer, die zur Entfernung von gebundenem Wasser aus der Heteropolysäure-Komponente des geträgerten Heteropolysäure-Katalysators ausreicht; und
(iii) während des Haltens des geträgerten Heteropolysäure-Katalysators von Schritt (ii) bei einer Temperatur von mindestens 220 °C, Inkontaktbringen des geträgerten Heteropolysäure-Katalysators mit dem Reaktantenzulauf mit einer Temperatur von mindestens 220 °C.

2. Verfahren nach Anspruch 1, wobei in Schritt (ii), der wärmebehandelte geträgerte Heteropolysäure-Katalysator von Schritt (i) für mindestens eine Stunde bei einer Temperatur von mindestens 220 °C gehalten wird.

3. Verfahren nach Anspruch 1 oder Anspruch 2, wobei Schritt (iii) in zwei Schritten durchgeführt wird:
(iiia) während des Haltens des geträgerten Heteropolysäure-Katalysators von Schritt (ii) bei einer Temperatur von mindestens 220 °C, Inkontaktbringen des geträgerten Heteropolysäure-Katalysators mit Wasser mit einer Temperatur von mindestens 220 °C; und
(iiib) während des Haltens des geträgerten Heteropolysäure-Katalysators von Schritt (iiia) bei einer Temperatur von mindestens 220 °C, Eintragen des Oxygenatreaktanten in das Wasser von Schritt (iiia) zur Bildung des Reaktantenzulaufs.

4. Verfahren nach einem der Ansprüche 1 bis 3, wobei der/die Oxygenatreaktant(en) ein Alkohol und/oder Alkoholderivat ist/sind.

5. Verfahren nach Anspruch 4, wobei der/die Oxygenatreaktant(en) Ethanol und/oder ein Ethanolderivat ist/sind.

6. Verfahren nach Anspruch 5, wobei der Oxygenatreaktant Ethanol ist.

7. Verfahren nach einem der Ansprüche 1 bis 6, wobei der geträgerte Heteropolysäure-Katalysator ein geträgerter Silicowolframsäure-Katalysator ist.

8. Verfahren nach Anspruch 7, wobei der geträgerte Heteropolysäure-Katalysator ein geträgerter 12-Wolframatokieselsäure-Katalysator ist.

9. Verfahren nach einem der Ansprüche 1 bis 8, wobei die Menge von Heteropolysäure in dem geträgerten Heteropolysäure-Katalysator im Bereich von 10 Gew.-% bis 50 Gew.-% bezogen auf das Gesamtgewicht des geträgerten Heteropolysäure-Katalysators liegt.

10. Verfahren nach einem der Ansprüche 1 bis 9, wobei das Verfahren zur Herstellung eines Alkens aus einem Oxygenat bei einer Temperatur im Bereich von 180 °C bis 270 °C durchgeführt wird.

11. Verfahren nach einem der Ansprüche 1 bis 10, wobei das Verfahren zur Herstellung eines Alkens aus einem Oxygenat bei einem Druck im Bereich von 0,1 MPa bis 4,5 MPa durchgeführt wird.

12. Verfahren nach einem der Ansprüche 1 bis 11, wobei vor Schritt (i) des Verfahrens der geträgerte Heteropolysäure-Katalysator bevorzugt durch Erwärmen des geträgerten Heteropolysäure-Katalysators auf eine Temperatur von mindestens 220 °C und Leiten von Dampf über den erwärmten geträgerten Heteropolysäure-Katalysator behandelt wird, gefolgt durch Erwärmen des mit Dampf behandelten geträgerten Heteropolysäure-Katalysators auf eine Temperatur von mindestens 220 °C unter einer wasserfreien Atmosphäre.

13. Verfahren nach Anspruch 12, wobei der geträgerte Heteropolysäure-Katalysator vorher in einem Verfahren zur Herstellung eines Alkens aus einem Oxygenat eingesetzt wurde.

14. Verfahren zur Behandlung eines geträgerten Heteropolywolframsäure-Katalysators zur Verwendung bei der Herstellung eines Alkens aus einem Oxygenat, umfassend die Schritte:
(a) Erwärmen des geträgerten Heteropolywolframsäure-Katalysators auf eine Temperatur von mindestens 220 °C und Leiten von Dampf über den genannten geträgerten Heteropolywolframsäure-Katalysator; und
(b) Erwärmen des gemäß Schritt (a) behandelten geträgerten Heteropolywolframsäure-Katalysators auf mindestens 220 °C in einer wasserfreien Atmosphäre.

15. Verfahren nach Anspruch 14, wobei Schritt (b) direkt nach Schritt (a), während des Haltens des Katalysators bei einer Temperatur von mindestens 220 °C durchweg während des gesamten Verfahrens, durchgeführt wird.

## Revendications

1. Procédé de préparation d'un alcène à partir d'un composé oxygéné, comprenant la mise en contact d'un flux d'alimentation réactif comprenant au moins un composé oxygéné réactif et de l'eau avec un catalyseur hétéropolyacide supporté à une température d'au moins 170 °C, le procédé étant amorcé en utilisant une procédure de démarrage comprenant les étapes suivantes :
(i) le chauffage du catalyseur hétéropolyacide supporté à une température d'au moins 220 °C ;
(ii) le maintien du catalyseur hétéropolyacide supporté soumis à un traitement thermique de l'étape (i) à une température d'au moins 220 °C pendant suffisamment de temps pour éliminer l'eau liée du constituant hétéropolyacide du catalyseur hétéropolyacide supporté ; et
(iii) tout en maintenant le catalyseur hétéropolyacide supporté de l'étape (ii) à une température d'au moins 220 °C, la mise en contact du catalyseur hétéropolyacide supporté avec le flux d'alimentation réactif ayant une température d'au moins 220 °C.

2. Procédé selon la revendication 1, dans lequel à l'étape (ii), le catalyseur hétéropolyacide supporté soumis à un traitement thermique de l'étape (i) est maintenu à une température d'au moins 220 °C pendant au moins une heure.

3. Procédé selon la revendication 1 ou la revendication 2, dans lequel l'étape (iii) est effectuée en deux étapes :
(iiia) tout en maintenant le catalyseur hétéropolyacide supporté de l'étape (ii) à une température d'au moins 220 °C, la mise en contact du catalyseur hétéropolyacide supporté avec de l'eau ayant une température d'au moins 220 °C ; et
(iiib) tout en maintenant le catalyseur hétéropolyacide supporté de l'étape (iiia) à une température d'au moins 220 °C, l'introduction du composé oxygéné réactif dans l'eau de l'étape (iiia) pour former le flux d'alimentation réactif.

4. Procédé selon l'une quelconque des revendications 1 à 3, dans lequel le ou les composés oxygénés réactifs sont des alcools et/ou des dérivés d'alcools.

5. Procédé selon la revendication 4, dans lequel le ou les composés oxygénés réactifs sont de l'éthanol et/ou des dérivés d'éthanol.

6. Procédé selon la revendication 5, dans lequel le composé oxygéné réactif est de l'éthanol.

7. Procédé selon l'une quelconque des revendications 1 à 6, dans lequel le catalyseur hétéropolyacide supporté est un catalyseur acide silicotungstique supporté.

8. Procédé selon la revendication 7, dans lequel le catalyseur hétéropolyacide supporté est un catalyseur acide 12-tungstosilicique.

9. Procédé selon l'une quelconque des revendications 1 à 8, dans lequel la quantité d'hétéropolyacide dans le catalyseur hétéropolyacide supporté est de 10 % en poids à 50 % en poids relativement au poids total du catalyseur hétéropolyacide supporté.

10. Procédé selon l'une quelconque des revendications 1 à 9, dans lequel le procédé de préparation d'un alcène à partir d'un composé oxygéné est effectué à une température de 180 °C à 270 °C.

11. Procédé selon l'une quelconque des revendications 1 à 10, dans lequel le procédé de préparation d'un alcène à partir d'un composé oxygéné est effectué à une pression de 0,1 MPa à 4,5 MPa.

12. Procédé selon l'une quelconque des revendications 1 à 11, dans lequel, avant l'étape (i) du procédé, le catalyseur hétéropolyacide supporté est préférablement traité par chauffage du catalyseur hétéropolyacide supporté à une température d'au moins 220 °C et passage de vapeur par-dessus le catalyseur hétéropolyacide supporté chauffé, avec ensuite un chauffage du catalyseur hétéropolyacide supporté traité par la vapeur à une température d'au moins 220 °C sous une atmosphère anhydre.

13. Procédé selon la revendication 12, dans lequel le catalyseur hétéropolyacide supporté a été utilisé auparavant dans un procédé de préparation d'un alcène à partir d'un composé oxygéné.

14. Procédé de traitement d'un catalyseur acide hétéropolytungstique supporté destiné à une utilisation dans la préparation d'un alcène à partir d'un composé oxygéné, comprenant les étapes suivantes :
(a) le chauffage du catalyseur acide hétéropolytungstique supporté à une température d'au moins 220 °C et le passage de vapeur par-dessus ledit catalyseur acide hétéropolytungstique supporté ; et
(b) le chauffage du catalyseur acide hétéropolytungstique supporté traité selon l'étape (a) à une température d'au moins 220 °C dans une atmosphère anhydre.

15. Procédé selon la revendication 14, dans lequel l'étape (b) est effectuée directement après l'étape (a) tout en maintenant le catalyseur à une température d'au moins 220 °C pendant toute la durée du procédé.
